# EUROPEAN PATENT SPECIFICATION

(11) **EP 3 929 275 B1**
(45) Date of publication and mention of the grant of the patent: **05.11.2025**
(21) Application number: 20759901.0
(22) Date of filing: 17.02.2020
(51) Int. Cl.: C12M 1/00, C12M 3/06, C12M 1/12

(54) **CELL CULTURE CHIP**
ZELLKULTUR-CHIP
PUCE DE CULTURE CELLULAIRE

(30) Priority: 22.02.2019 JP 2019030374
(43) Date of publication of application: 29.12.2021
(73) Proprietor: Ushio Denki Kabushiki Kaisha, Tokyo 100-8150 (JP)
(72) Inventor: HIROSE,Kenichi, Tokyo 100-8150 (JP); KOYANAGI,Hiroshi, Tokyo 100-8150 (JP)
(74) Representative: Maiwald GmbH
(86) International application number: PCT/JP2020/005980
(87) International publication number: WO 2020/171001

(56) References cited:
- EP-A1- 3 279 310
- WO-A1-2017/074959
- JP-A- 2005 027 598
- JP-A- 2006 325 537
- JP-A- 2018 009 924
- JP-A- 2018 083 294
- US-A1- 2017 002 315

## Description

### TECHNICAL FIELD

The present invention relates to a cell culture chip.

### BACKGROUND ART

Conventionally, a culture of cells and tissues has been performed using culture dishes or plates as agar or media. Because the culture of cells and tissues using these culture dishes and plates is performed in a two-dimensional (planar) environment, an extracellular microenvironment cannot be reproduced. Therefore, in recent years, there has been proposed a cell culture chip (also referred to as a "biochip" or a "microchip") having a microchannel that can produce a three-dimensional (stereoscopic) cell culture/experimental environment, which has been difficult by the conventional method.

For example, Patent Document JP-A-2001-38811 discloses a resin structure that can be used for the cell culture chip. EP 3 279 310 A1 discloses a cell culture apparatus having embedded microchannels which is made of an assembly of different layers stacked upon each other and fixed by a holder.

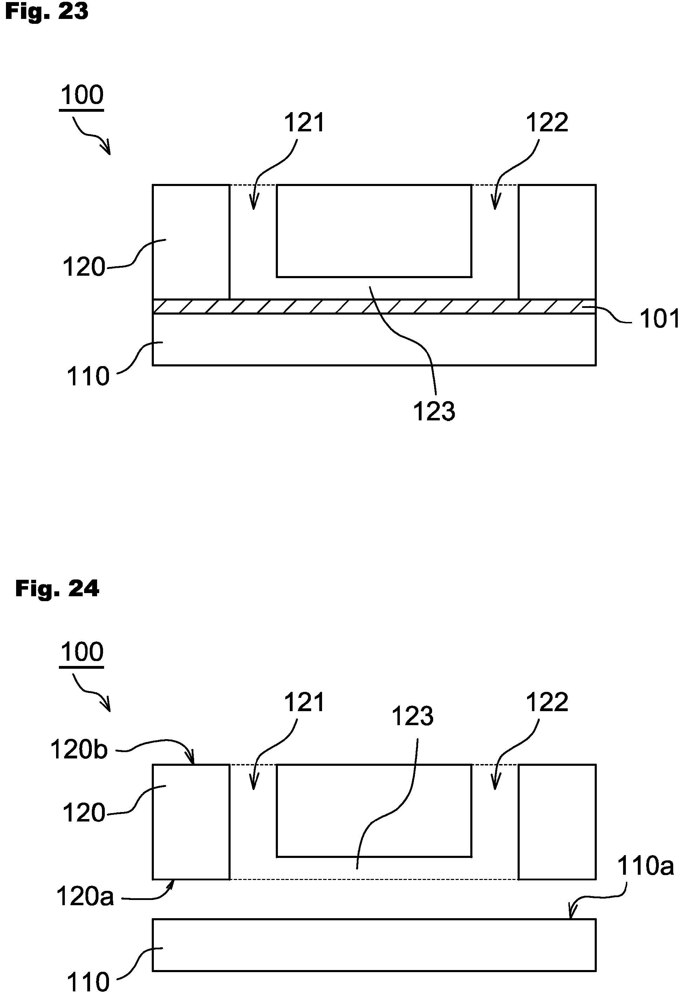

Fig. 23 is a cross-sectional view schematically showing a resin structure (hereinafter, referred to as a "cell culture chip") disclosed in Patent Document 1. The cell culture chip 100 includes a first substrate 110 and a second substrate 120 and is formed by bonding these two substrates together. Both the first substrate 110 and the second substrate 120 are made of polymethyl methacrylate (acrylic resin). Fig. 24 is a view schematically illustrating a state before the two substrates (110, 120) are bonded together.

The first substrate 110 is formed in a flat plate shape. The second substrate 120 is formed with a recess 123 for constituting a space for culturing cells and openings (121, 122) for constituting a channel of a culture solution. More specifically, the recess 123 is formed on a surface 120a side of the second substrate 120. The opening 121 and the opening 122 are each formed to be exposed at one end on a side of a surface 120b of the second substrate 120 and penetrate the second substrate 120 to reach the recess 123.

At the time of bonding, first, a thin film 101 made of polymer material is applied onto one surface 110a of the first substrate 110 by a spin coating method. Thereafter, under a heating environment, the surface 120a of the second substrate is pressed against the surface 110a of the first substrate 110 to which the thin film 101 is applied. The thin film 101 functions as a bonding agent for bonding the first substrate 110 to the second substrate 120.

When the first substrate 110 and the second substrate 120 are bonded together, the recess 123 forms a space (culture space) sandwiched between the substrates (110, 120). That is, a channel (microchannel) is formed by the openings (121, 122) and the recess 123. When the culture solution is flowed in from one of the openings (121, 122), the cells can be cultured in the culture space.

### SUMMARY OF THE INVENTION

### PROBLEMS TO BE SOLVED BY THE INVENTION

There is a case in which cells are cultured while culture conditions are variously changed to verify the states of the cells. Conventionally, cell culture chips different for each culture condition have been used. In recent years, cell culture chips that can support various measurements such as simultaneous measurement of a plurality of specimens and multiple measurements with different measurement methods have been studied. From the viewpoint of forming many microchannels in one cell culture chip, one cell culture chip becomes larger than the conventional one. The conventional cell culture chip has a size of about 25 mm × 75 mm in length and width. On the other hand, such a large cell culture chip has, for example, a size of about 85 mm × 128 mm in length and width, and a thickness of each of two substrates constituting the microchip is, for example, several mm or more.

As described above, it is necessary to form the recess 123 for forming the channel in the second substrate 120 which is one of the two substrates (110, 120) to be bonded together. The second substrate 120 having the recess 123 and the openings (121, 122) as such can be produced by injection molding.

However, in the case of the large second substrate 120 described above being produced by injection molding, the second substrate 120 after molding tends to warp easily. In addition, because the second substrate 120 having a shape provided with the recess 123 and the openings (121, 122) is produced by injection molding, at the time of pouring a resin, molds are pressed against each other so that the resin does not flow into a region where the recess 123 and the openings (121, 122) are formed. For this reason, there is a case in which a streak-like pattern called a weld line is formed at a portion where the resin that has passed around avoiding the mold is slightly cooled and then reassociated. For this reason, there is a case in which a large unevenness called undulation is generated on the surface of the second substrate 120.

If the second substrate 120 in a warped or undulated state is bonded to the first substrate 110 as described above, there is a possibility that a bonding failure occurs or bubbles enter depending on the locations. In addition, the height (vertical length of recess 123 on a paper surface in Fig. 23) of the microchannel may change depending on the location. The state as such is not preferable because a flow rate and a reaction rate of a specimen to be circulated through the microchannel deviate from an expected design value, due to such as culture conditions becoming different for each culture space in the case of culturing the cells.

Furthermore, when the second substrate 120 is bonded to the first substrate 110 in the state of being warped as a whole, the position of the height of the microchannel may vary depending on the location. In a case in which the cells are cultured in a plurality of microchannels (culture spaces) mounted on one cell culture chip, the state of the cells cultured in each culture space is continuously observed (photographed). At this time, if a height position of a bottom surface of the channel varies depending on locations, at the time of photographing the entire cell culture chip, it is necessary to focus on each culture space, and the work becomes complicated.

Given the above problems, it is an object of the present invention to provide a cell culture chip in which a plurality of culture spaces are mounted, the cell culture chip having less warpage and suppressing a change in height positions of the culture spaces according to locations as compared with the conventional art.

### MEANS FOR SOLVING THE PROBLEMS

A cell culture chip according to the present invention includes a bottom substrate and a base body part bonded onto the bottom substrate.

The base body part includes:
a first surface and a second surface opposing the first surface;
a plurality of first portions configured to form a culture space and dispersedly arranged at a plurality of places in a direction parallel to the first surface; and
a cavity that penetrates the base body part from the first surface and reaches the second surface, in a region where the first portions are not formed.

The plurality of first portions each include:
a recessed region formed to extend in the direction parallel to the first surface on a side of the first surface; and
a plurality of opening grooves formed to penetrate the base body part from a plurality of places in the recessed region and reach the second surface.

The cavity is configured such that at least a part of an end in the direction parallel to the first surface is positioned inside an outer edge of the base body part.

The bottom substrate and the first surface of the base body part are bonded together to form the culture space in which the recessed region is sandwiched between the bottom substrate and the base body part.

Because the base body part has the cavity reaching from the first surface to the second surface, high flexibility is generated in the base body part. At the time of bonding the base body part and the bottom substrate together, both surfaces (the first surface and the second surface) of the base body part are substantially divided into a plurality of subdivisions by the cavity. That is, the action of deformation caused by pressing a certain subdivision at the time of bonding is less likely to affect the other subdivisions. As a result, even if undulation or warpage has occurred in the base body part due to injection molding because the entire surface can be pressed with a uniform pressure by a pressing process of when the base body part is bonded to the bottom substrate, the undulation or warpage can be eliminated.

Furthermore, because the cavity is formed to penetrate the base body part, an upper surface of the bottom substrate can be visually recognized through the cavity when the base body part and the bottom substrate are overlapped with each other. This facilitates alignment when the base body part and the bottom substrate are bonded together.

Furthermore, according to this configuration, because the cavity after the bottom substrate and the base body part are bonded together remains as a groove reaching the upper surface of the bottom substrate from an upper surface side of the base body part, there is an effect that a liquid such as water can be stored in the groove for adjusting the humidity of the culture space.

The base body part can be made of a resin material such as polymethyl methacrylate (PMMA), polycarbonate (PC), cycloolefin copolymer (COC), cycloolefin polymer (COP), polystyrene (PS), silicone, and acrylic. In addition to the resin material, the bottom substrate may be made of a glass material such as quartz glass.

The base body part may be constituted of a base body substrate having a rectangular shape and being a substrate different from the bottom substrate,
when viewed from a direction orthogonal to the first surface, the plurality of first portions may be arranged in a matrix, respectively aligned in a first direction parallel to a first side part constituting the outer edge of the base body part and in a second direction parallel to a second side part constituting the outer edge and being different from the first side part, and
the cavity may be formed to extend in the first direction at a position between regions where the plurality of first portions are arranged in the first direction.

In this case, both surfaces (the first surface and the second surface) of the base body part are substantially divided into a plurality of subdivisions in the second direction by the cavity formed by extending in the first direction.

The cavity may be formed to extend in the first direction at a position outside, in the second direction, a region where the plurality of first portions is arranged in the first direction.

Further, the cavity may have one end in the first direction that reaches the first side part. As a result, the flexibility of the base body part is further improved, and the warpage and deflection can be eliminated by a smaller pressing force by the pressing process at the time of bonding.

The plurality of opening grooves may include a first opening groove and a second opening groove formed at a position spaced apart from the first opening groove in the second direction, and
the recessed region may be formed to extend in the second direction to connect the first opening groove to the second opening groove.

When the base body part is produced by injection molding, the resin is poured into the region where the opening groove is formed, from both sides in a state of molds being pressed against each other. Therefore, the resin flows while avoiding the region where the molds are pressed against each other. At this time, there is a case in which the resin passes around the place of the mold and then reassociates together to form a streak-like pattern called a weld line.

According to the above configuration, while the extending direction of the cavity is the first direction, the extending direction of the recessed region, that is, the separating direction of the two opening grooves (the first opening groove and the second opening groove) connected to the recessed region is the second direction. That is, the extending direction of the cavity is different from the extending direction of the recessed region. As described above, because the cavity is formed to penetrate the first surface and the second surface, when the base body part including the cavity is produced by injection molding, the resin flows in a state in which the region where the cavity is formed is also pressed by the two molds. That is, the resin flows from a side of the second side part in the extending direction of the cavity, that is, in the first direction.

In this case, because the mold is pressed against the place where the opening groove (the first opening groove and the second opening groove) is formed, the resin that has passed around avoiding the mold may reassociate at a position shifted in the first direction to the opening groove. Because this position is a position shifted in a direction parallel to the first surface to the recessed region constituting the culture space, even if the weld line occurs, the observation of the cultured cells is hardly affected.

On the other hand, for example, in a case in which the extending direction of the recessed region is set to the first direction which is the same as the extending direction of the cavity, the resin that has flowed while avoiding the portion of the mold for forming the first opening groove may reassociate at a position on a side of the second opening groove to the first opening groove. This position is a region where the recessed region is to be formed in a direction parallel to the first surface. Therefore, when the weld line is generated at the above position, the recessed region constituting the culture space overlaps with the weld line when viewed from a direction orthogonal to the first surface, and there is a possibility that the observation of the cultured cells becomes difficult.

The base body part may include:
an outer edge having a rectangular frame shape and including first side parts facing each other and second side parts facing each other;
a connecting part that connects the first side parts facing each other by extending in a second direction parallel to the second side parts; and
an island part having a plate shape including the first surface and the second surface, the island part is provided in plural numbers and the plurality of island parts being dispersedly arranged at a plurality of positions between the connecting part and the outer edge in a state of being connected to a part of the connecting part,
the first portion may be formed in the island part, and
the cavity may be formed in a region enclosed by the outer edge and the connecting part at a position outside the island part.

In the case of such a configuration, by the first portion formed in the island part being bonded to the bottom substrate, the recessed region included in the first portion constitutes the culture space. Further, at the position outside the island part, the cavity penetrating the entire body is formed, and the base body part is substantially divided into a plurality of subdivisions by each island part. Therefore, also in this configuration, the action of deformation caused by pressing at the time of bonding to a certain subdivision (island part) hardly affects the other subdivisions (island parts). As a result, even if undulation or warpage has occurred in the base body part due to injection molding because the entire surface can be pressed with a uniform pressure by the pressing process at the time of bonding to the bottom substrate, the undulation or warpage can be eliminated.

Furthermore, according to this configuration, the cavity is formed at the position outside the island part, enclosed by the outer edge and the connecting part. Therefore, when the bottom substrate and the base body part are bonded together, the groove is formed outside the first portion (that is, the culture space) including the opening groove and the recessed region, enclosed by the outer edge, the connecting part, and the bottom substrate. According to such a configuration, there is an effect that a liquid such as water can be stored in this groove for adjusting the humidity of the culture space.

The base body part may include a plurality of the first connecting parts formed spaced apart in a first direction parallel to the first side part, and
the island part may be formed at a position enclosed by the connecting parts adjacent to each other in the first direction.

The plurality of opening grooves included in the first portion formed in the island part may include a first opening groove and a second opening groove formed at a position spaced apart from the first opening groove in the second direction, and
the recessed region included in the first portion formed in the island part may be formed to extend in the second direction to connect the first opening groove to the second opening groove.

### EFFECT OF THE INVENTION

According to the present invention, it is possible to realize a cell culture chip that, even while a plurality of culture spaces are mounted, has less warpage and suppresses a change in height positions of the culture spaces according to locations as compared to the conventional art.

### BRIEF DESCRIPTION OF THE DRAWINGS

Fig. 1 is a plan view schematically showing a structure according to a first embodiment of a cell culture chip of the present invention.
Fig. 2A is a cross-sectional view taken along a line A1-A1 in Fig. 1.
Fig. 2B is a cross-sectional view taken along a line A2-A2 in Fig. 1.
Fig. 3 is a view illustrating a bottom substrate and a base body part in Fig. 2A separately.
Fig. 4 is a partially enlarged view of Fig. 1.
Fig. 5 is a plan view schematically showing a structure of the base body part before being bonded to the bottom substrate in the cell culture chip of the first embodiment.
Fig. 6 is a schematic cross-sectional view illustrating, in the same manner as Fig. 2A, the cell culture chip into which a culture solution is injected.
Fig. 7 is a view schematically showing, in the same manner as Fig. 5, a shape of a mold used when the base body part is produced by injection molding.
Fig. 8 is a view schematically showing a flow of a resin during the injection molding.
Fig. 9 is a view schematically showing, in the same manner as Fig. 5, another shape of the mold used when the base body part is produced by the injection molding.
Fig. 10 is a plan view schematically showing another structure of the cell culture chip according to the first embodiment of the present invention.
Fig. 11 is a cross-sectional view taken along a line A3-A3 in Fig. 10.
Fig. 12 is a plan view schematically showing another structure of the cell culture chip according to the first embodiment of the present invention.
Fig. 13 is a plan view schematically showing still another structure of the cell culture chip according to the first embodiment of the present invention.
Fig. 14 is a plan view schematically showing still another structure of the cell culture chip according to the first embodiment of the present invention.
Fig. 15 is a plan view schematically showing a structure of a cell culture chip according to a second embodiment of the present invention.
Fig. 16A is a cross-sectional view taken along a line B1-B1 in Fig. 15.
Fig. 16B is a cross-sectional view taken along a line B2-B2 in Fig. 15.
Fig. 17 is a plan view schematically showing a structure of a base body part before being bonded to a bottom substrate in the cell culture chip of the second embodiment.
Fig. 18 is a view schematically showing, in the same manner as Fig. 5, a shape of a mold used when the base body part included in the cell culture chip of the second embodiment is produced by injection molding.
Fig. 19 is a view schematically showing a flow of a resin during the injection molding.
Fig. 20 is a plan view schematically showing a structure of a first portion of a cell culture chip according to another embodiment.
Fig. 21 is a plan view schematically showing a structure of a first portion of a cell culture chip according to another embodiment.
Fig. 22 is a plan view schematically showing a structure of a cell culture chip of another embodiment.
Fig. 23 is a cross-sectional view schematically showing a structure of a conventional cell culture chip.
Fig. 24 is a view illustrating two substrates included in the cell culture chip illustrated in Fig. 23 in a separated manner.

### MODE FOR CARRYING OUT THE INVENTION

A cell culture chip according to the present invention is described referring to the drawings. Note that the following drawings are only schematically illustrated. That is, dimensional ratios in the drawings do not necessarily coincide with actual dimensional ratios, and the dimensional ratios do not necessarily coincide with each other between the drawings.

### [First embodiment]

A first embodiment of a cell culture chip according to the present invention is described.

### < <Structure> >

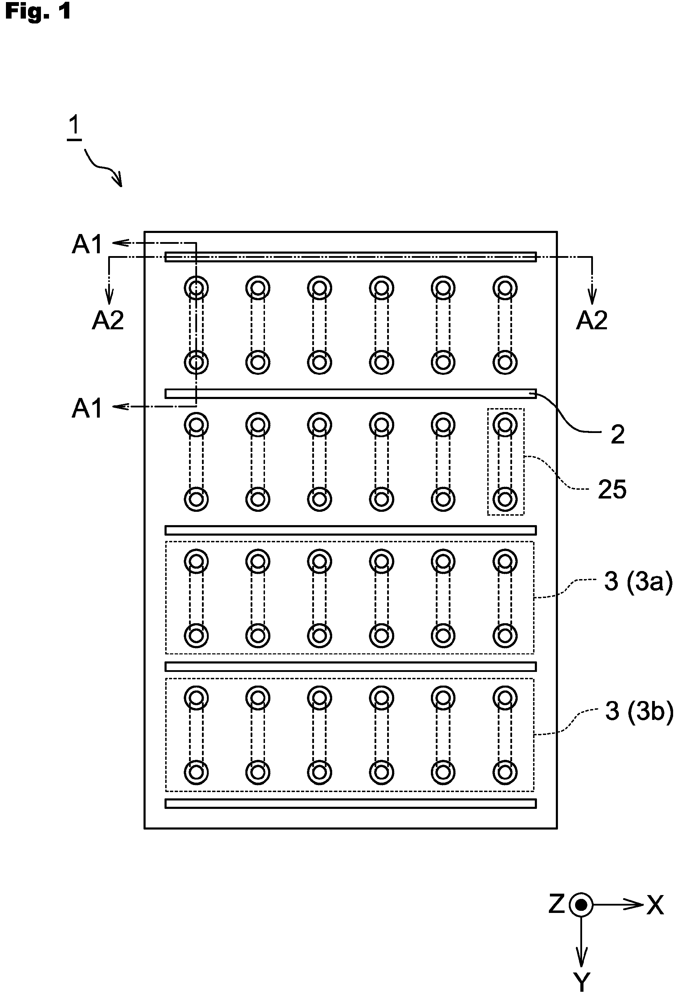

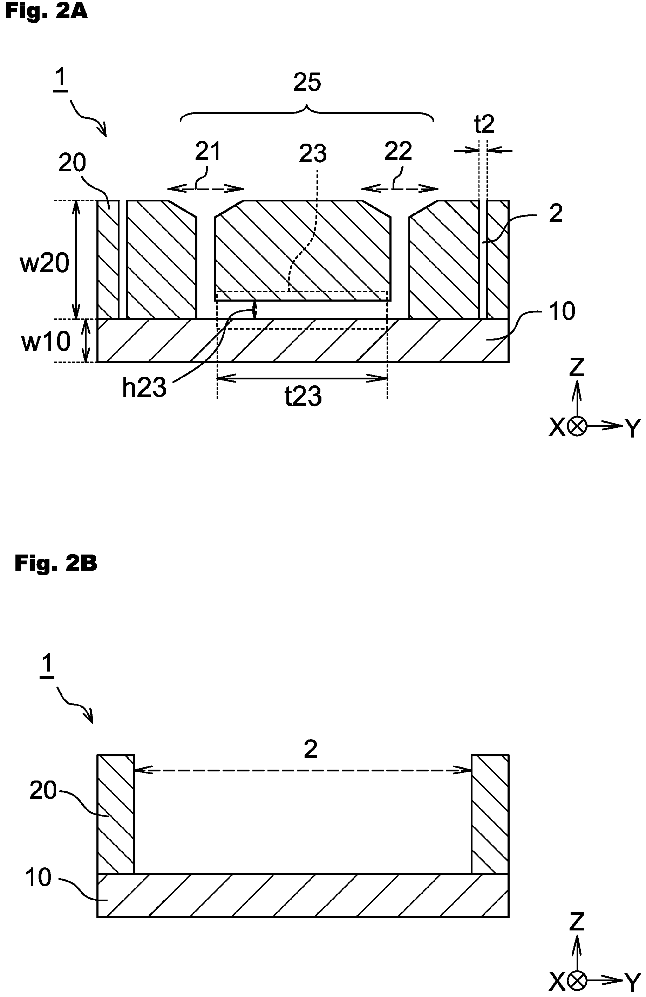

Fig. 1 is a plan view schematically showing a structure of the cell culture chip according to the present embodiment. Fig. 2A is a cross-sectional view taken along a line A1-A1 in Fig. 1, and Fig. 2B is a cross-sectional view taken along a line A2-A2 in Fig. 1.

A cell culture chip 1 includes a bottom substrate 10 and a base body part 20. As illustrated in Figs. 2A and 2B, the base body part 20 is bonded to an upper surface of the bottom substrate 10. In the following description, a surface on which the bottom substrate 10 and the base body part 20 are bonded together is defined as an XY plane, and a direction orthogonal to the XY plane is defined as a Z-direction.

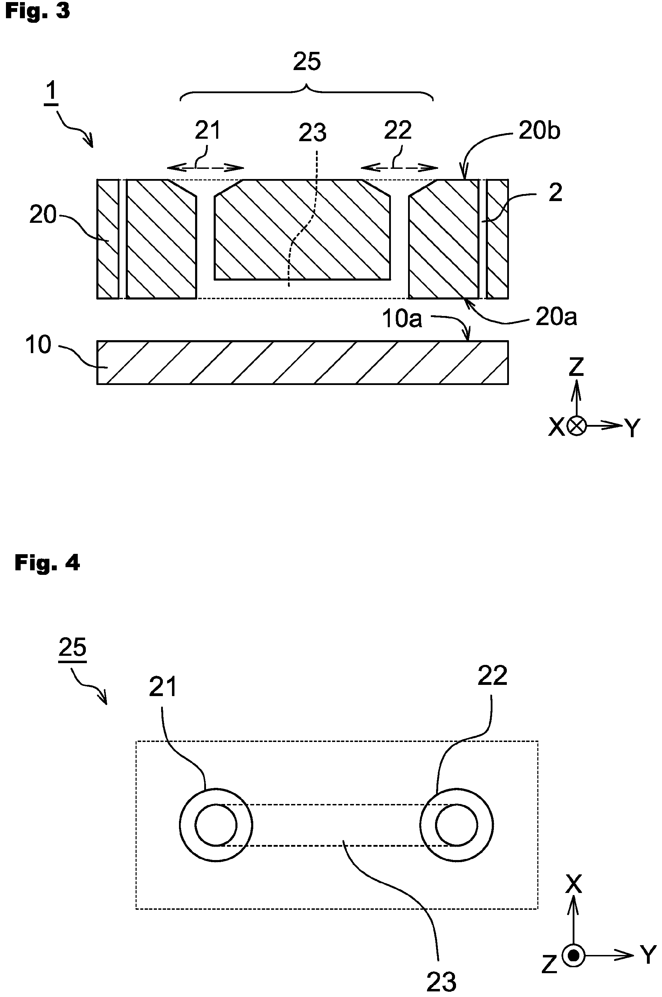

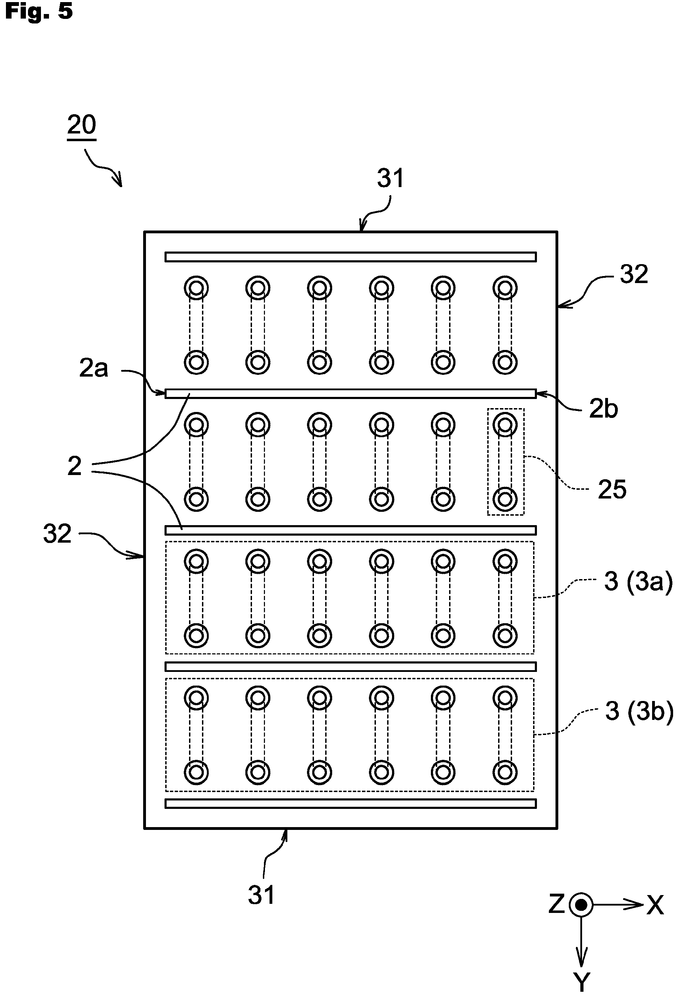

Fig. 3 is a view illustrating the bottom substrate 10 and the base body part 20 in Fig. 2A separately. Fig. 4 is an enlarged view of a region indicated by a reference numeral 25 in Fig. 1. This region corresponds to a "first portion 25" described later. Fig. 5 is a schematic plan view of the base body part 20 before being bonded to the bottom substrate 10 as viewed from an upper surface (the surface opposite to a bonding surface). In Fig. 5, an outer edge of the base body part 20 is denoted by a thick line to emphasize the outer edge.

Hereinafter, the detailed structure of the cell culture chip 1 is described referring to Figs. 1 to 5.

The base body part 20 has a substrate shape in which the outer edge is constituted of a pair of first side parts 31 parallel to an X-direction and a pair of second side parts 32 parallel to a Y-direction. That is, in the present embodiment, the base body part 20 constitutes a "base body substrate". The X-direction corresponds to a "first direction", and the Y-direction corresponds to a "second direction".

The base body part 20 has slit-shaped cavities 2 extending in the X-direction. The cavities 2 are formed at a plurality of places spaced apart in the Y-direction. Further, the base body part 20 has, in each of subdivisions 3 partitioned by the cavities 2 formed at positions spaced apart in the Y-direction, a plurality of culture space forming regions each including opening grooves (21, 22) and a recessed region 23. Hereinafter, this region is referred to as a "first portion 25". The opening groove 21 corresponds to a "first opening groove", and the opening groove 22 corresponds to a "second opening groove".

In the present embodiment, as shown in Figs. 1 and 5, the plurality of first portions 25 are arranged spaced apart in the X-direction in the same subdivision 3 (3a). Furthermore, the plurality of first portions 25 are also arranged spaced apart in the X-direction in the different subdivision 3 (3b) spaced apart in the Y-direction. That is, in the present embodiment, the plurality of first portions 25 are formed in a matrix shape in the base body part 20 in a state of being aligned in the X-direction and the Y-direction.

As described above, the first portion 25 includes the opening grooves (21, 22) and the recessed region 23. As shown in Fig. 3, the opening grooves (21, 22) are through holes penetrating the base body part 20 in the Z-direction and are formed at different positions on the XY plane. That is, one end of the opening groove 21 and one end of the opening groove 22 are both exposed at different positions on a second surface 20b of the base body part 20.

Further, the recessed region 23 is a recess formed on a side of a first surface 20a of the base body part 20 and is connected to the opening groove 21 and the opening groove 22. That is because the recessed region 23 does not penetrate the base body part 20 unlike the opening grooves (21, 22), the recessed region 23 is not exposed on a side of the second surface 20b of the base body part 20. In Figs. 1, 4, and 5, the recessed region 23 each are indicated by a broken line to indicate that the recessed region 23 is not exposed to the side of the second surface 20b of the base body part 20.

As shown in Fig. 3, the opening grooves (21, 22) each have a shape having an inclined surface whose opening area decreases as the groove advances from the side of the second surface 20b to the side of the first surface 20a. However, the shape illustrated in Fig. 3 is merely an example, and various shapes can be adopted. For example, the opening grooves (21, 22) may have the same opening area between the second surface 20b and the first surface 20a in the Z-direction.

In the present embodiment, the opening groove 21 and the opening groove 22 constituting the same first portion 25 are formed at positions spaced apart in the Y-direction. The recessed region 23 has a shape extending in the Y-direction to communicate the opening groove 21 and the opening groove 22 formed at positions spaced apart in the Y-direction.

As shown in Fig. 5, in the present embodiment, both ends (2a, 2b) of the cavity 2 are located inside the second side part 32. As described above, the cavity 2 penetrates the base body part 20 from the first surface 20a to reach the second surface 20b. That is, the cavity 2 forms a slit-shaped groove extending in the X-direction on the inner side of the outer edge of the base body part 20 (see Figs. 2B and 5).

The base body part 20 is made of a resin material that can be injection-molded. More specifically, the base body part 20 is made of a resin material such as polymethyl methacrylate (PMMA), polycarbonate (PC), cycloolefin copolymer (COC), cycloolefin polymer (COP), polystyrene (PS), silicone, and acrylic. On the other hand, the bottom substrate 10 may be made of a glass material such as quartz glass other than the above resin material. The substrate 20 and the bottom substrate 10 are preferably both made of materials exhibiting light transmittance, from the viewpoint of ease of observation of cells in culture.

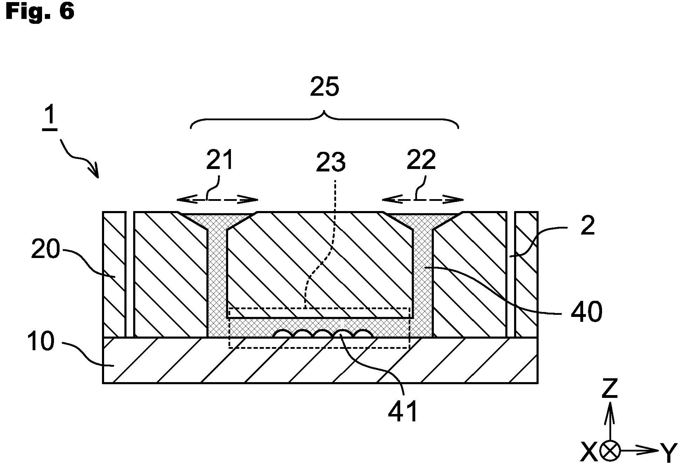

By bonding the first surface 20a of the base body part 20 and a surface 10a of the bottom substrate 10 together, the recessed region 23 forms a tubular space sandwiched between the base body part 20 and the bottom substrate 10 (see Fig. 2A). As described above, the recessed region 23 is communicated with the opening grooves (21, 22). As a result, a culture space is formed by the opening grooves (21, 22) and the recessed region 23. For example, by injecting a culture solution 40 containing cells 41 from the opening groove 21 or the opening groove 22, as shown in Fig. 6, the cells 41 can be cultured in the recessed region 23 constituting the culture space.

An example of dimensions is as follows (see Fig. 2A). A height (thickness) w10 of the bottom substrate 10 is about 1 mm, and preferably 100 µm or more and 2 mm or less. A height w20 of the base body part 20 is about 3 mm, which corresponds to the depth of the opening grooves (21, 22). A height h23 of the recessed region 23 (culture space) is about 300 µm, and preferably 200 µm or more and 500 µm or less. A length t23 of the recessed region 23 in the Y-direction (longitudinal direction) is about 9 mm. In addition, the volume of the space from the opening groove 21 through the recessed region 23 and reaching the opening groove 22 is 100 mm³ (100 µL) or less, and more preferably 10 mm³ (10 µL).

A width t2 (slit width) of the cavity 2 in the Y direction is 0.5 mm or more and 5 mm or less, and preferably 1 mm or more and 3 mm or less. In a case in which the slit width t2 of the cavity 2 is smaller than 0.5 mm, there is a possibility that molds are deformed by the pressure during injection molding, and a gap is generated between the molds. This is not preferable because the gap as such generates burrs of the resin. On the other hand, in a case in which the slit width t2 of the cavity 2 is larger than 5 mm, the slit width t2 becomes too large for the resin to flow smoothly. Accordingly, there is a risk of bubbles and residual strain being generated to cause a warpage of the base body part 20.

The size of the bottom substrate 10 and the base body part 20 in the XY plane is optional, as long as it conforms to a predetermined standard. As an example, according to dimensions conforming to American National Standards Institute (ANSI)/the Society for Laboratory Automation and Screening (SLAS) standard number 1-2004, the size of the bottom substrate 10 and the base body part 20 in the XY plane is 85 mm × 128 mm.

### < <Method of manufacturing> >

Next, an example of a method of manufacturing the above-described cell culture chip 1 is described.

### (Step S1: forming of the base body part 20 and the bottom substrate 10)

The bottom substrate 10 having a rectangular flat plate shape is prepared. In addition, the base body part 20 having a rectangular flat plate shape, and in which the cavities 2 and the first portions 25 each including the opening grooves (21, 22) and the recessed region 23 are formed is produced by injection molding.

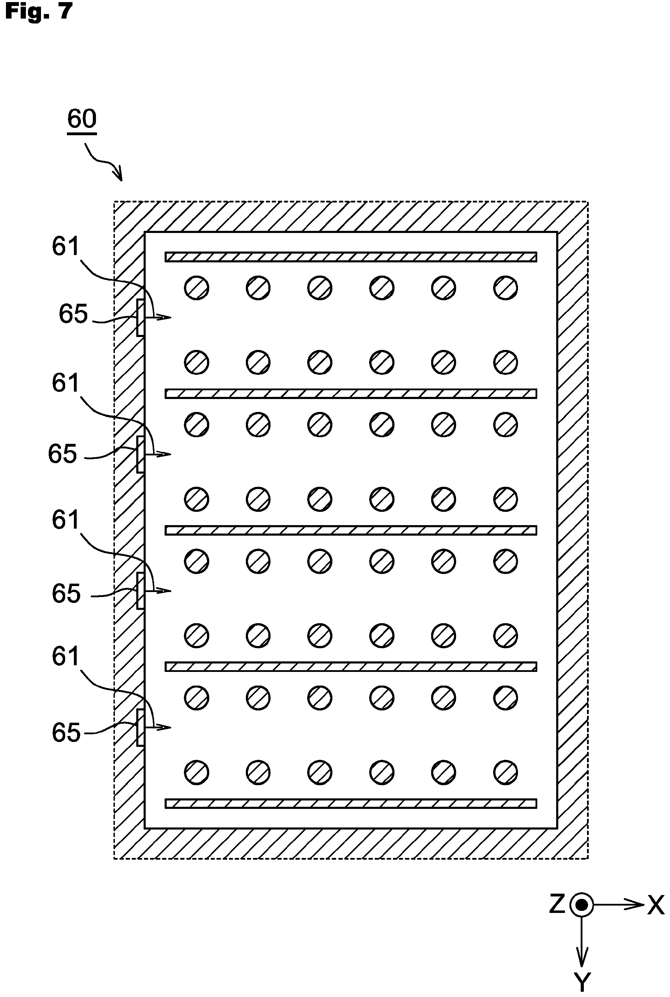

The base body part 20 is produced, for example, by pouring molten resin into mold being arranged in a region where the cavities 2 and the first portions 25 are to be formed. Fig. 7 schematically illustrates, in the same manner as Fig. 5, a shape of a mold 60 used when the base body part 20 is produced by injection molding. In Fig. 7, the mold 60 is positioned in hatched regions.

In the example shown in Fig. 7, resin 61 is injected in the X-direction from a place corresponding to the number of subdivisions 3 partitioned by the cavities 2. In Fig. 7, a reference numeral 65 denotes a part of the mold into which the resin 61 is injected, and corresponds to a place constituting a gate. That is, the example shown in Fig. 7 shows a case in which the resin 61 is injected by a so-called side gate method.

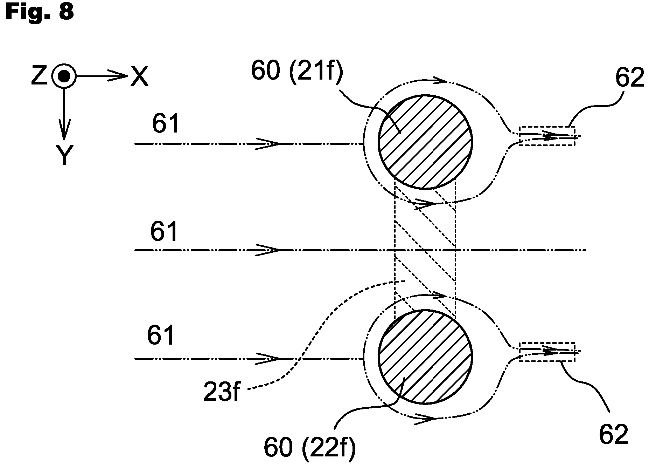

Fig. 8 is a diagram schematically showing a flow of the resin. As described above, unlike the opening grooves (21, 22), the recessed region 23 is formed only in a part in the depth direction (Z-direction) of the base body part 20. For the convenience of explanation, Fig. 8 schematically illustrates the flow of the resin on the XY plane at the position of the Z-coordinate where the recessed region 23 does not exist. Additionally, for explanatory purposes, Fig. 8 illustrates by broken lines and hatching a region 23f where the recessed region 23 is to be formed. Further, in Fig. 8, a part of the mold 60 is positioned in a region 21f where the opening groove 21 is to be formed, and another part of the mold 60 is positioned in a region 22f where the opening groove 22 is to be formed.

As shown in Fig. 8, because there is the mold 60 for forming the opening grooves (21, 22), the resin 61 traveling in the X direction travels to avoid a place where the mold 60 is positioned. At this time, there is a region where streams of the resin 61 that has passed through the position of the mold 60 merges (a region 62). In this region 62, there is a possibility that a streaky pattern called a weld line is formed.

As described above, in the cell culture chip 1 of the present embodiment, the two opening grooves (21, 22) constituting the same first portion 25 are spaced apart in the Y-direction. Therefore, as shown in Fig. 8, the molds (21f, 22f) for forming the two opening grooves (21, 22) are also arranged at positions spaced apart in the Y-direction. When the resin 61 is injected from the X direction in this state, the resin merging region 62 is formed in a region between the opening grooves (21, 22), that is, outside the recessed region 23. As a result, the formation of the weld line at the position on the XY plane where the recessed region 23 is formed is suppressed.

If the weld line is formed at the same position as the position on the XY plane where the recessed region 23 is formed, it may interfere with the observation of the cell 41 in culture. In addition, if the weld line is formed to communicate with the recessed region 23, there is a possibility that the culture solution 40 flows out to a weld line side, and the environment of the culture space changes and adversely affects the cells 41, which is not preferable.

However, as described above, by injecting the resin 60 from a direction different from the spaced-apart direction of the opening grooves (21, 22) (this is also the extending direction of the recessed region 23), it is possible to avoid and suppress the formation of the weld line at the same position on the XY plane as the recessed region 23.

In the above, the description is made of the case in which injection molding is performed using the side gate method in which the resin 61 is injected from gates 65 arranged at a plurality of places on a side of the side part (more specifically, the side of the second side part 32) of the base body part 20 to be a molded object. However, when the base body part 20 of the present invention is produced, the gate used is not limited to the side gate method. For example, a film gate method or a tab gate method in which the resin 61 is injected from the side of the entire second side part 32 may be adopted.

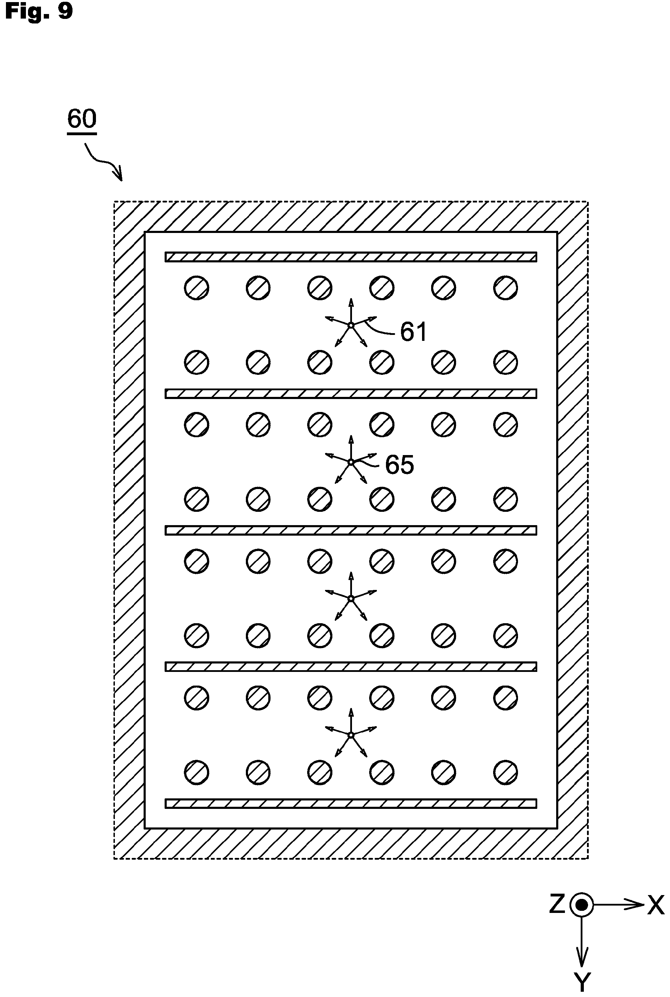

For example, as illustrated in Fig. 9, it is also possible to use a so-called pin gate method, in which the resin 61 is injected from the gates 65 discretely arranged at predetermined positions in the region between the cavities 2. In the case of adopting the pin gate method, it is preferable to install the gate 65 near the center of the position between the cavities 2, and to inject the resin 61.

### (Step S2: bonding of the base body part 20 and the bottom substrate 10 together)

The base body part 20 produced in step S1 and the bottom substrate 10 are bonded together. Specifically, the following procedure is performed.

First, the bonding surfaces (10a, 20a) of the base body part and the bottom substrate are subjected to a treatment for activating the surfaces. As a method of surface activation treatment, a method of irradiating ultraviolet rays or a method of bringing plasma gas into contact can be used. This surface activation treatment is performed to change the surface 10a of the bottom substrate 10 and the first surface 20a of the base body part 20 to a state in which a terminal is substituted with a hydroxy group (OH group) to be in a state suitable for bonding.

The ultraviolet irradiation method is performed by irradiating the first surface 20a of the base body part 20 and the surface 10a of the bottom substrate 10 with a vacuum ultraviolet ray having a wavelength of 200 nm or less from an ultraviolet light source such as a xenon excimer lamp having an emission line at a wavelength of 172 nm. As another example of the ultraviolet light source, a low-pressure mercury lamp having an emission line at 185 nm and a deuterium lamp having an emission line in a wavelength range of 120 to 200 nm can be suitably used. The illuminance of the vacuum ultraviolet ray is, for example, 10 to 100 mW/cm², and the irradiation time is, for example, 10 to 60 seconds.

The method of bringing the plasma gas into contact is performed by generating a plasma of a process gas containing such as nitrogen gas and argon gas as main components and containing 0.01 to 5 vol% of oxygen gas by atmospheric pressure plasma and bringing the plasma into contact with the first surface 20a of the base body part 20 and the surface 10a of the bottom substrate 10. It is also possible to use a mixed gas of nitrogen gas and clean dry air (CDA). The contact time of the plasma gas is, for example, 5 to 100 seconds.

Next, the base body part 20 and the bottom substrate 10 are overlapped with each other in a state in which the bonding surfaces (10a, 20a) of the two subjected to the surface activation treatment are in contact with each other, and the two are pressed and pressurized.

A pressurization condition is appropriately set according to the materials constituting the base body part 20 and the bottom substrate 10 and a heating temperature. A magnitude of a pressing force only needs to be within a range in which the recessed region 23 is not excessively deformed, which is, for example, 1 to 10 MPa, and a pressing time is, for example, 60 to 600 seconds.

In this pressing process, from the viewpoint of strengthening the bonding, a stacked body including the base body part 20 and the bottom substrate 10 may be heated simultaneously with and/or after the pressurization. A heating condition for heating the stacked body is, for example, a heating temperature of 40 to 130°C and a heating time of 60 to 600 seconds.

Because the cavities 2 penetrating in the Z-direction are formed in the base body part 20 produced in step S1 described above, the base body part has high flexibility. Therefore, even if undulation or warpage has occurred in the base body part 20 produced in step S1, the first surface 20a of the base body part 20 is deformed along the surface 10a of the bottom substrate 10 by being pressurized and pressed in the bonding process in step S2, and the undulation or warpage is eliminated.

Further, because the cavities 2 are formed to extend in the X-direction while penetrating the base body part 20 in the Z-direction, the base body part 20 is substantially divided into the plurality of subdivisions 3 by the cavities 2. Therefore, in a certain subdivision 3a, even if the first surface 20a of the base body part 20 is deformed along the surface 10a of the bottom substrate 10 by being pressed, this deformation effect is less likely to spread to another subdivision 3b at the adjacent position through the cavity 2. As a result, the entire first surface 20a of the base body part 20 (a portion where the recessed regions 23 and the cavities 2 are not formed) can be bonded to the surface 10a of the bottom substrate 10 by a small pressurizing force that does not excessively deform the recessed region 23.

It has been confirmed that in a case in which the cavity 2 does not completely penetrate the base body part 20 in the Z direction, the base body part 20 is warped more largely than in the case in which the cavity 2 completely penetrates the base body part 20 in the Z direction. In addition, it has been confirmed that if the base body part 20 is pressed against the bottom substrate 10 to an extent of not causing cracking, the entire surface cannot be pressed with sufficient pressure, and only partial bonding can be performed.

As a result, the base body part 20 is bonded onto the surface of the bottom substrate 10, and the cell culture chip 1 shown in Figs. 1 to 2B is produced.

### <<Another configuration example>>

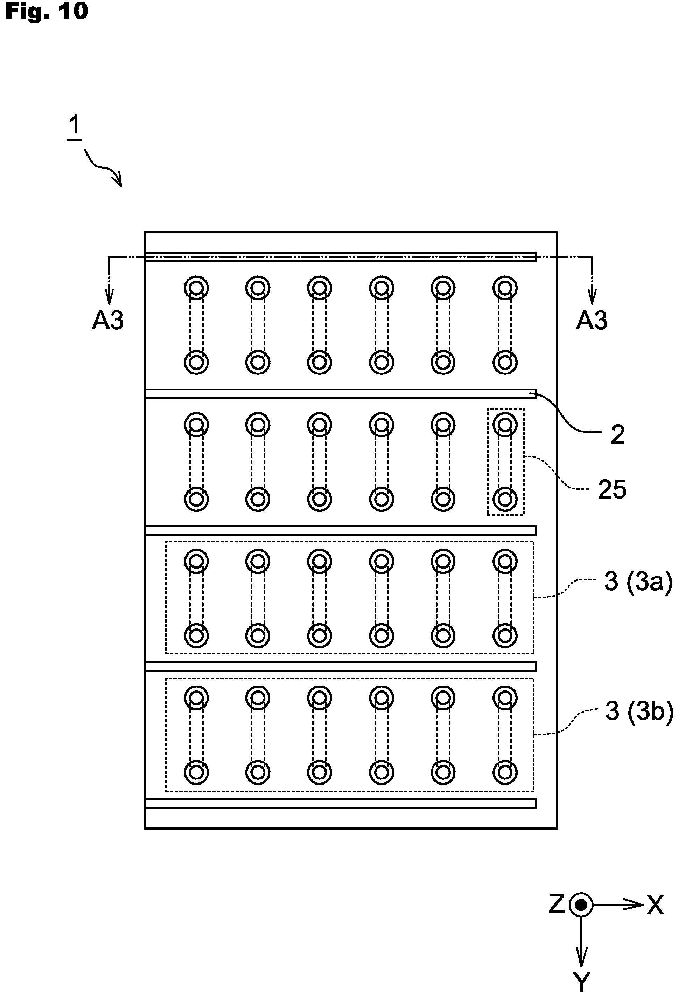

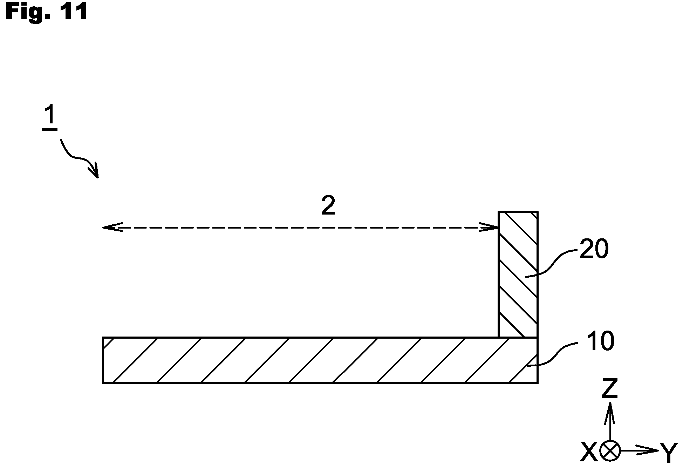

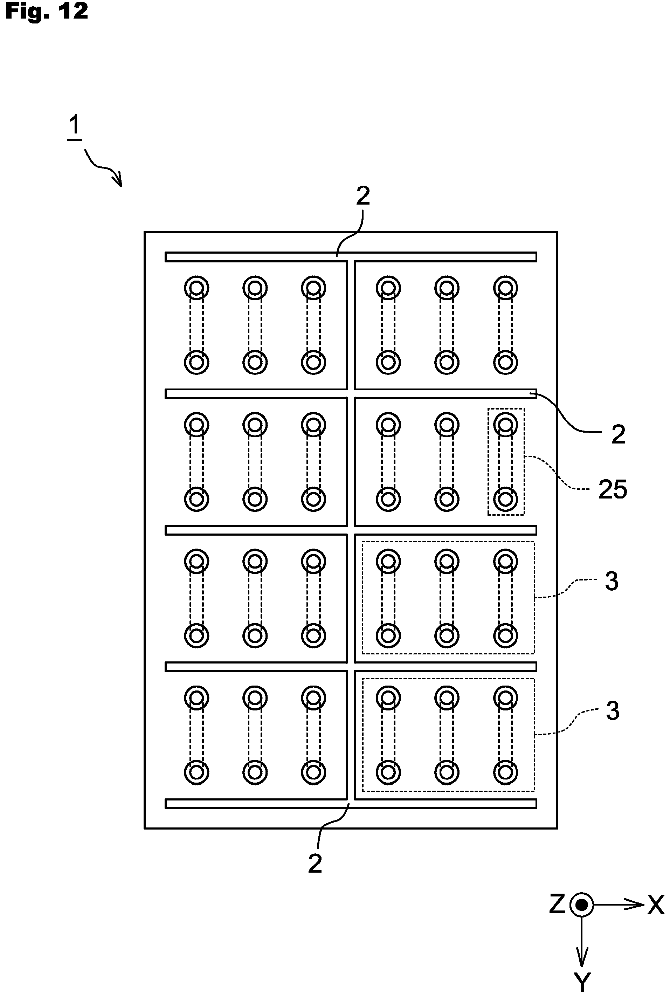

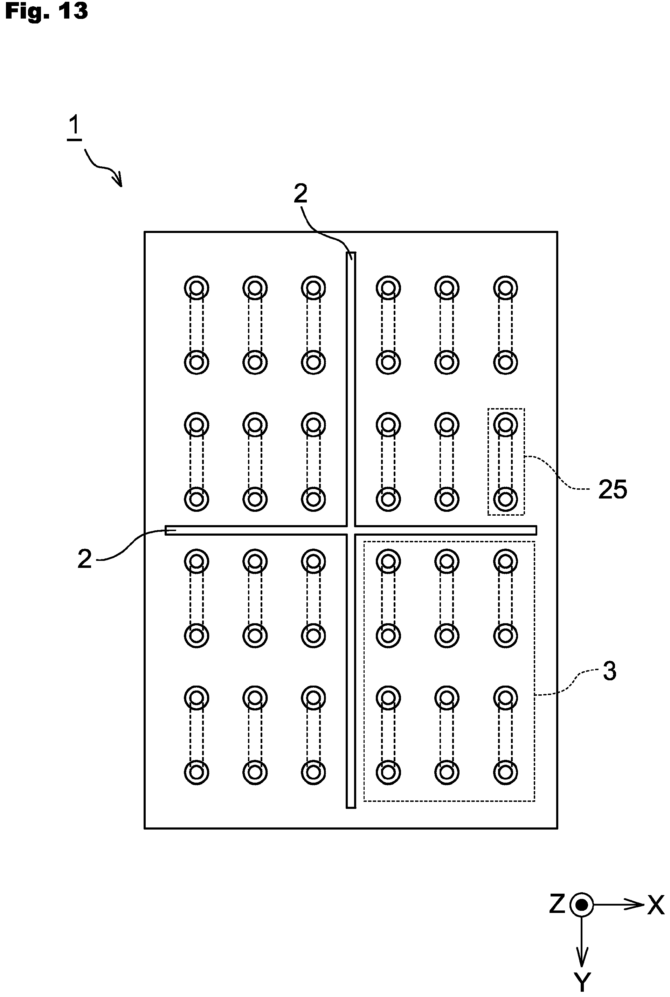

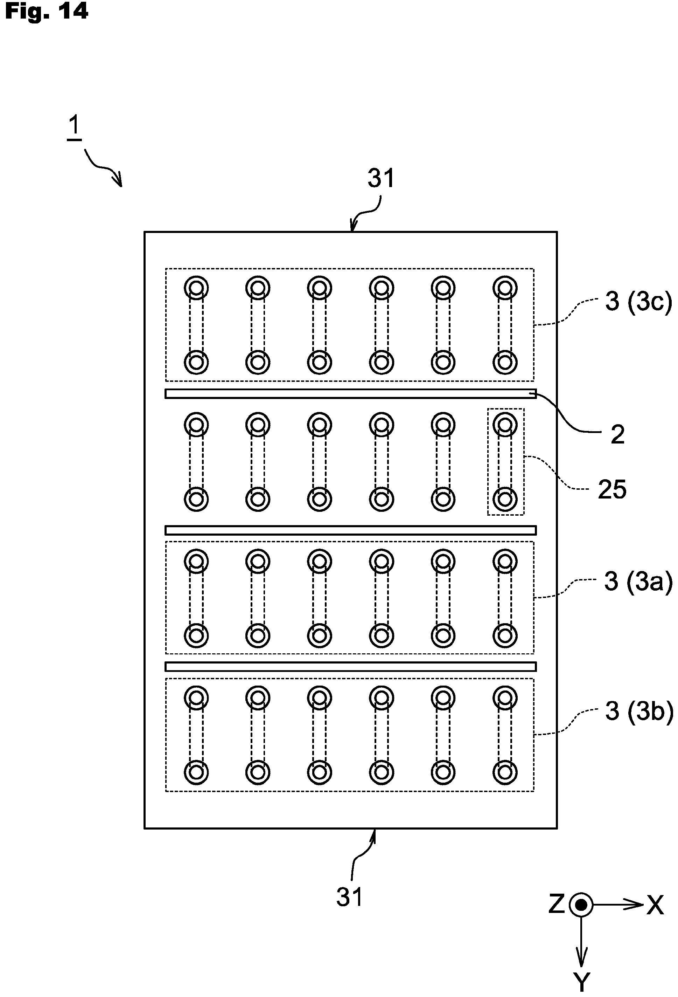

Another configuration example of the cell culture chip 1 of the present embodiment is described referring to the drawings.
<1> In the above embodiment, it has been described that both the ends of the cavity 2 extending in the X-direction are positioned inside the second side part 32. However, one end of each of the cavities 2 in the X-direction may reach the second side part 32. Fig. 10 illustrates, in the same manner as Fig. 1, a plan view of the cell culture chip 1 according to this another configuration and Fig. 11 corresponds to a cross-sectional view taken along a line A3-A3 in Fig. 10.
   According to this configuration, because one end of the cavity 2 in the X-direction reaches the second side part 32, the flexibility of the base body part 20 is further improved as compared with the configuration illustrated in Fig. 5. As a result, in the case of undulation or warpage occurring in the first surface 20a of the base body part 20, the force required in step S2 to press the base body part 20 against the surface of the bottom substrate 10 to eliminate the undulation or warpage can be further reduced.
<2> In the above embodiment, the case in which the cavities 2 have a shape extending in the X direction has been described, but as shown in Figs. 12 and 13, the cavities 2 may have a shape extending in both the X direction and the Y direction.
<3> It has been described that in the cell culture chip 1 shown in Fig. 1, all the subdivisions 3 are positioned in the region between the cavities 2 in the Y direction. However, as shown in Fig. 14, the subdivisions 3 (in the example of Fig. 14, the subdivision 3b and a subdivision 3c) located on outermost sides in the Y direction may be positioned in a region between the cavity 2 and the outer edge (the first side part 31).

### [Second embodiment]

A second embodiment of a cell culture chip according to the present invention is described focusing on points different from the first embodiment.

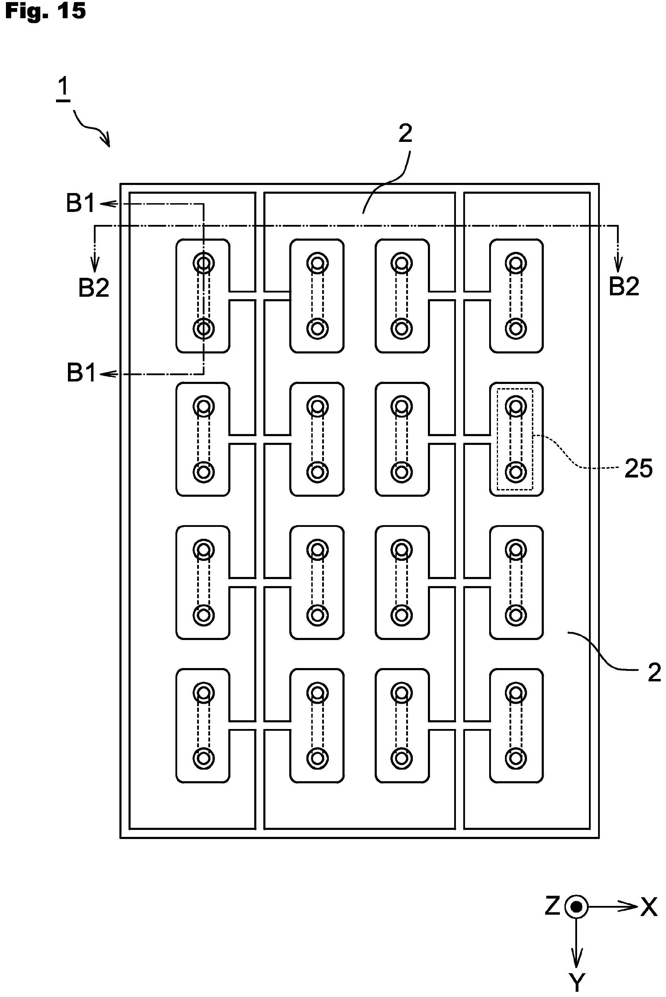

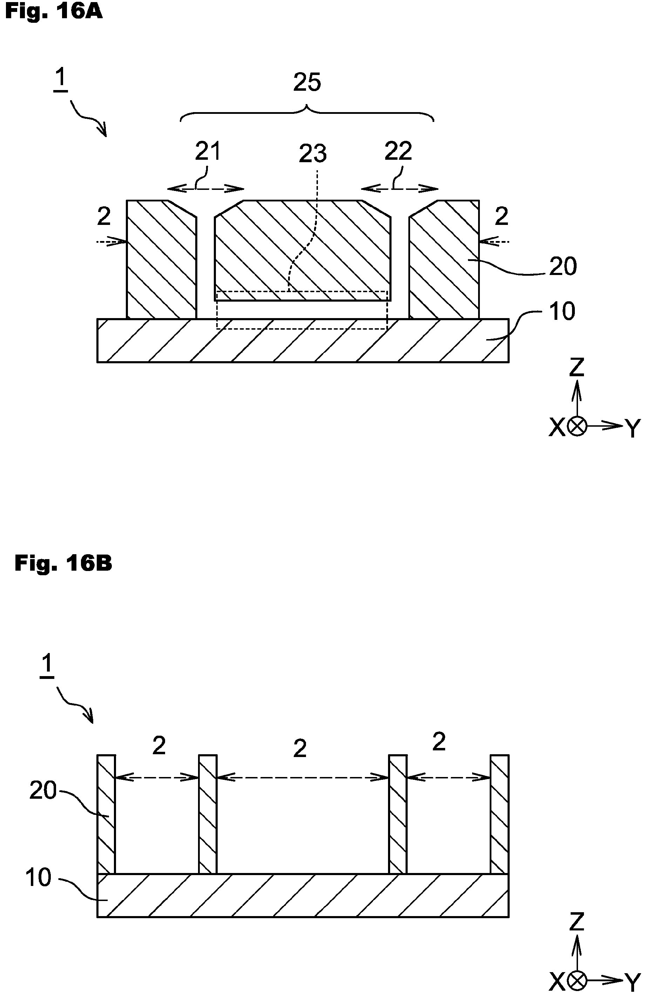

Fig. 15 is a plan view schematically showing a structure of the cell culture chip according to the present embodiment. Fig. 16A is a cross-sectional view taken along a line B1-B1 in Fig. 15, and Fig. 16B is a cross-sectional view taken along a line B2-B2 in Fig. 15.

As in the first embodiment, a cell culture chip 1 of the present embodiment also includes a bottom substrate 10 and a base body part 20, and the base body part 20 is bonded to a surface of the bottom substrate 10. However, the cell culture chip 1 of the present embodiment is different from that in the first embodiment in the shape of the base body part 20.

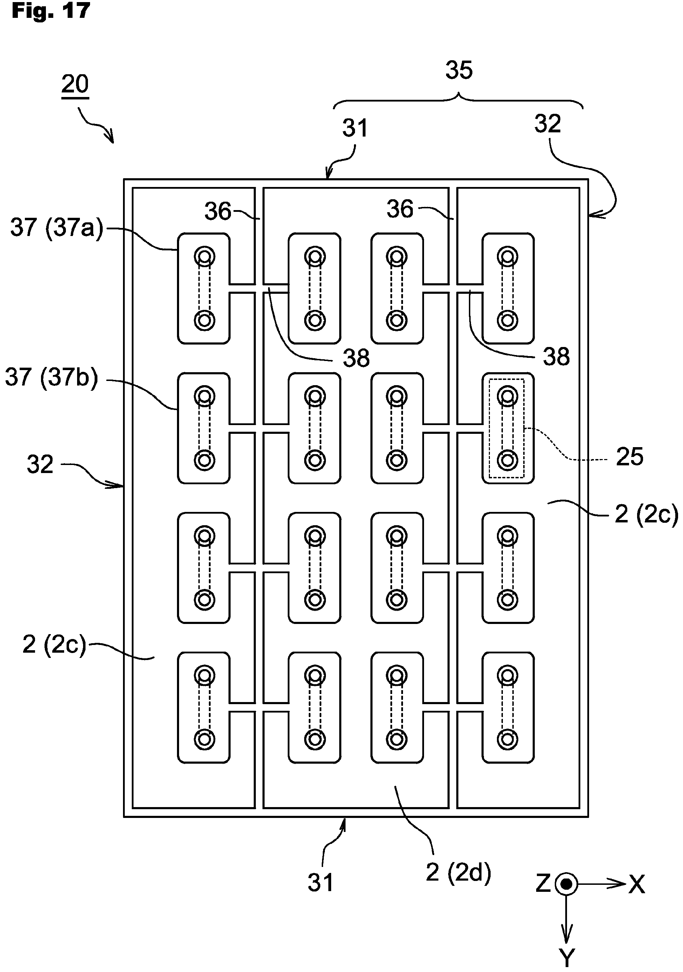

Fig. 17 is a schematic plan view of the base body part 20 before being bonded to the bottom substrate 10 as viewed from an upper surface (Z direction). As shown in Fig. 17, the base body part 20 includes an outer edge 35, connecting parts 36, and island parts 37.

The outer edge 35 has a frame shape including a pair of first side parts 31 parallel to the X-direction and a pair of second side parts 32 parallel to the Y-direction. In the example shown in Fig. 17, the connecting part 36 extends in the Y-direction (direction parallel to the second side parts 32) and connects the first side parts 31 facing each other. The island parts 37 each have a plate shape including a first surface 20a and a second surface 20b and are dispersedly arranged at a plurality of positions in a state of being connected to a part of the connecting part 36 at a position between the connecting part 36 and the outer edge 35. In the example shown in Fig. 17, the plurality of island parts 37 is formed in a matrix shape in a state of being aligned in the X-direction and the Y-direction.

As shown in Fig. 16A, a cross-sectional view taken along the line B1-B1 of the cell culture chip 1 according to the present embodiment is substantially similar to the cross-sectional view taken along the line A1-A1 of the cell culture chip 1 according to the first embodiment shown in Fig. 2A. That is, the island part 37 is formed with a first portion 25 including opening grooves (21, 22) and a recessed region 23. Therefore, also in the present embodiment, the plurality of first portions 25 is formed in a matrix shape in the base body part 20 in a state of being aligned in the X direction and the Y direction.

In the cell culture chip 1 of the present embodiment, cavities 2 are formed in a region enclosed by the outer edge 35 and the connecting parts 36 at positions outside the island parts 37. More specifically, in the structure shown in Fig. 17, the cavities 2 includes cavities 2c formed in a region enclosed by the first side parts 31 facing each other, the second side part 32, and the connecting part 36, and a cavity 2d formed in a region enclosed by the first side parts 31 facing each other and the pair of connecting parts 36. Similar to the first embodiment, these cavities 2 (2c, 2d) are formed to penetrate the base body part 20 in the Z direction.

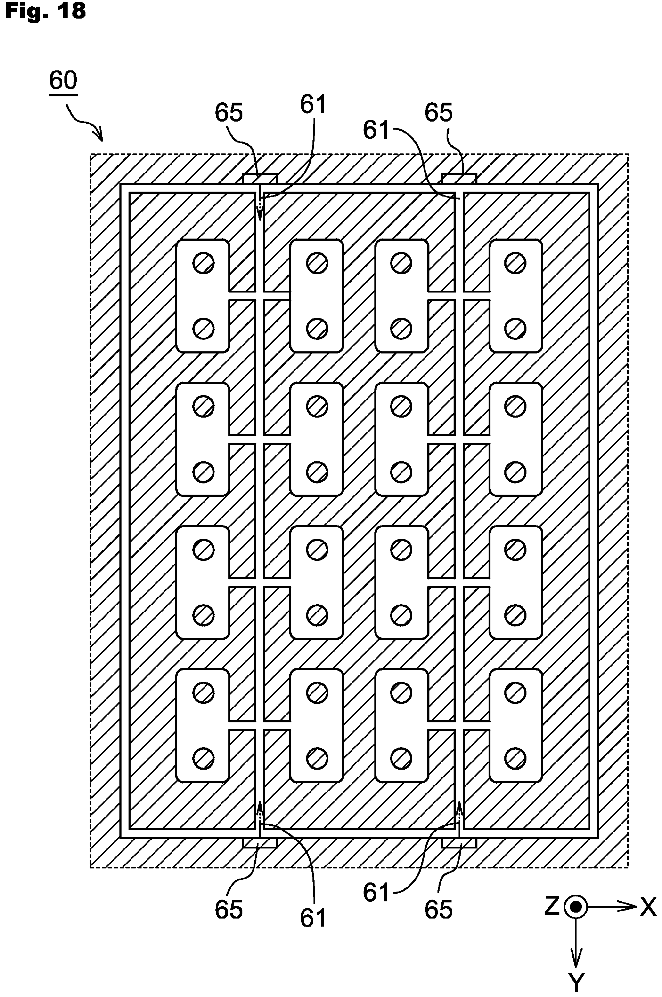

The base body part 20 having the shape as such can be manufactured by injection molding similarly to the first embodiment. At this time, it is preferable to inject resin 61 from the outside of a position at which a place where the connecting part 36 is formed intersects a place where the outer edge 35 (the first side part 31 in this case) is formed (from gates 65 in Fig. 18). Fig. 18 schematically illustrates, in the same manner as Fig. 17, a shape of a mold 60 used when the base body part 20 of the present embodiment is produced by injection molding.

As illustrated in Fig. 17, the opening grooves (21, 22) formed in the island part 37 are spaced apart in the Y-direction, and the recessed region 23 connecting the opening grooves (21, 22) also extends in the Y-direction. In addition, the island part 37 and the connecting part 36 formed by extending in the Y-direction are connected through a branch connecting part 38 branched in the X-direction from the connecting part 36.

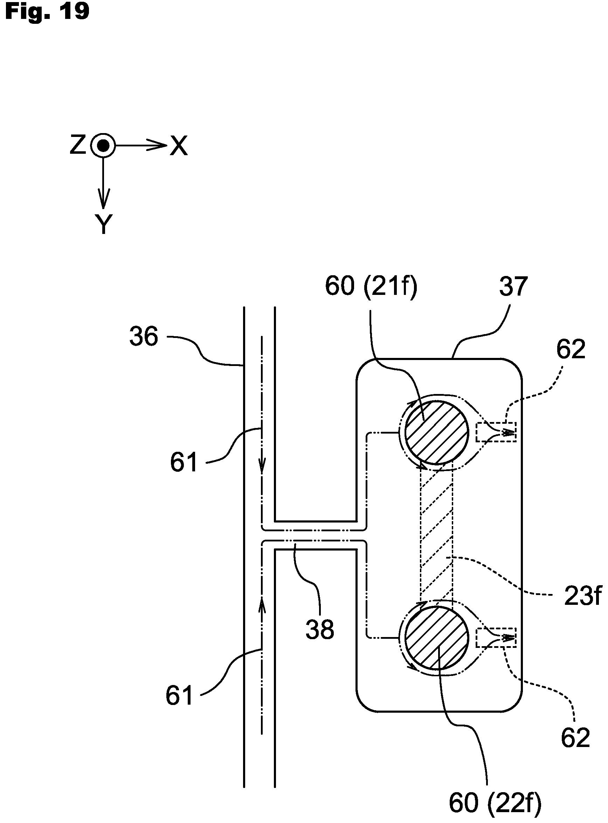

Under the above shape, when the resin 61 is injected from the gates 65, the resin 61 flows in the Y-direction along a region (runner portion) where the connecting parts 36 are formed. Fig. 19 is a view schematically showing, in the same manner as Fig. 8, a flow of the resin 61. In Fig. 19, for convenience of explanation, a region 23f where the recessed region 23 is to be formed is illustrated by broken lines and hatching. Further, in Fig. 19, a part of the mold 60 is positioned in a region 21f where the opening groove 21 is to be formed, and another part of the mold 60 is positioned in a region 22f where the opening groove 22 is to be formed.

As illustrated in Fig. 19, because the mold 60 for forming the opening grooves (21, 22) is present, the resin 61 traveling in the Y direction along the region where the connecting part 36 is formed travels in the X direction along the region where the branch connecting part 38 is formed, and then travels to avoid the place where the mold 60 is positioned. At this time, there is a possibility that the weld line is formed in a region 62 where streams of the resin 61 that has passed through the position of the mold 60 merges.

However, also in the present embodiment, similarly to the first embodiment, because the resin 61 is injected from the X-direction through the region where the branch connecting part 38 is to be formed in a state of the two openings (21, 22) being spaced apart in the Y-direction, the weld line is formed outside a region between the opening grooves (21, 22), that is, the recessed region 23. As a result, the formation of the weld line at the position on the XY plane where the recessed region 23 is formed is suppressed.

After the base body part 20 having the shape shown in Fig. 17 is produced, the bottom substrate 10 and the base body part 20 are bonded together by the same method as in the first embodiment. The island parts 37 constituting the first portions 25 are merely connected by the branch connecting part 38 and the connecting part 36 which have a thin width. That is, in the cell culture chip 1 of the present embodiment, the base body part 20 is substantially divided by the plurality of island parts 37. Therefore, in a certain island part 37a, even if the first surface 20a of the base body part 20 is deformed along the surface 10a of the bottom substrate 10 by being pressed, this deformation effect is less likely to spread to another island part 37b at the adjacent position through the cavity 2. As a result, the entire surface 20a of the base body part 20 (a portion where the recessed region 23 and the cavity 2 are not formed) can be bonded to the surface 10a of the bottom substrate 10 by a small pressurizing force that does not excessively deform the recessed region 23.

However, in the cell culture chip 1 of the present embodiment, the gates 65 may be arranged on a side of the second side part 32, and the resin 61 may be injected in the X-direction.

In the present embodiment, the wide cavity 2 is formed in the base body part 20 outside the island parts 37 where the first portions 25 forming the culture space are positioned. The cavity 2 has a groove shape that is surrounded by the base body part 20 and the bottom substrate 10, after the bottom substrate 10 and the base body part 20 are bonded together. Therefore, to adjust the humidity of the atmosphere at the time of culturing cells 41, a liquid such as water can be stored in this region.

[Examples] The point that warpage is suppressed and a large bonding area between the bottom substrate 10 and the base body part 20 can be secured according to the cell culture chip according to the present invention is described referring to examples. However, the present invention is not limited to the aspects of the examples.

As Example 1, Comparative Example 1, and Reference Example 1, the bottom substrate 10 and the base body part 20 having the following dimensions were prepared.
- Bottom substrate 10: (X) 85 mm × (Y) 128 mm × (Z) 1 mm ZEONEX 460R manufactured by Zeon Corporation
- Base body part 20: (X) 85 mm × (Y) 128 mm × (Z) 3 mm ZEONEX 460R manufactured by Zeon Corporation

In Example 1, the base body part 20 in which the cavities 2 each penetrating in the depth direction (Z-direction) with a slit width of 1 mm were formed was adopted. The length (X-direction) of the cavity 2 was 75 mm, and the number of cavities 2 arranged was seven. Further, six rows of the subdivisions 3 were arranged in the Y direction, and eight first portions 25 were arranged in the X direction in each subdivision 3.

Reference Example 1 is different from Example 1 in that the length in the depth direction of each of the cavities 2 provided in the base body part 20 was 2 mm. That is, the cavities 2 provided in the cell culture chip of Reference Example 1 did not penetrate the base body part 20 in the depth direction.

The cell culture chip of Comparative Example 1 is different from that of Example 1 in that the cavities 2 were not provided in the base body part 20.

Each of the bonding surfaces of the base body part 20 and the bottom substrate 10 of Example 1, Reference Example 1, and Comparative Example 1 was irradiated with an ultraviolet ray having a wavelength of 172 nm emitted from a xenon excimer lamp at an irradiance of 40 mW/cm² for 20 seconds. Thereafter, the bonding surfaces of the base body part 20 and the bottom substrate 10 of Example 1, Reference Example 1, and Comparative Example 1, respectively, were brought into contact with each other, and then pressing was performed at a pressure of 4 MPa for 300 seconds under a temperature environment of 90°C.

The results at this time are shown in Table 1.

**[Table 1]**

| | Slit | | Maximum warpage | Bonding area | Evaluation |
|---|---|---|---|---|---|
| | Width | Depth | | | |
| Example 1 | 1 mm | Penetrating | 0.5 mm | 100% | A |
| Comparative Example 1 | None | None | 0.5 mm | 20% | C |
| Reference Example 1 | 1 mm | 2 mm | 2.4 mm | 60% | C |

A method of measuring the bonding area is described.

When the base body part 20 and the bottom substrate 10 are bonded to each other, the base body part and the bottom substrate are integrated, so that an interface no longer exists at a portion where the two are bonded together without any gap. On the other hand, the interface remains at a place not completely bonded. Therefore, for each cell culture chip after bonding, a planar photograph was taken from a side of the bottom substrate 10, and the presence or absence of a dark part was verified. Because the portion where the interface is present is observed as the dark part as compared with the region where the interface is completely bonded, it is possible to determine whether or not the bonding is complete.

Specifically, for each cell culture chip after bonding, the planar photograph was taken from the side of the bottom substrate 10, image processing was performed on the taken image using image measurement software, and a ratio of an area of the dark part to a total area was calculated. By this ratio, a ratio of an actual bonding area to a total area to be bonded was calculated.

In the cell culture chip 1 of Example 1, the maximum warpage of the base body part 20 was suppressed to 0.5 mm, and the bonding area between the base body part 20 and the bottom substrate 10 was 100%. Therefore, the warpage was suppressed, and the entire surface was in surface contact, and thus it was confirmed that the cell culture chip was practically excellent. A bonding area of 95% or more between the base body part 20 and the bottom substrate 10 is considered to be within a practical range without problems. The evaluation "A" indicates that there is no practical problem.

In the cell culture chip of Comparative Example 1, the maximum warpage of the base body part 20 was suppressed to 0.5 mm, but the bonding area between the base body part 20 and the bottom substrate 10 was 20%. For this reason, the bottom substrate 10 and the base body part 20 were not sufficiently bonded together, and there is a problem in practical use as a cell culture chip. The evaluation "C" indicates that there is a problem in practical use.

In the cell culture chip of Reference Example 1, the bonding area between the base body part 20 and the bottom substrate 10 was 60%, and a bonding state was improved as compared with Comparative Example 1, but the maximum warpage of the base body part 20 was as large as 2.4 mm, and occurrence of a crack was observed at the portion of the slit. Therefore, there is a problem in practical use as a cell culture chip.

Also as can be seen from the above verification, by forming the slit (the cavity 2) in a state where the slit penetrates the base body part 20 in the Z direction, the base body part 20 and the bottom substrate 10 can be surface-bonded to each other while suppressing the warpage even after the process of bonding the base body part 20 and the bottom substrate 10 together.

### [Other embodiments]

Hereinafter, other embodiments are described.
<1> In each of the above embodiments, the cell culture chip 1 in which the pair of opening grooves (21, 22) is connected to the recessed region 235 constituting the culture space has been described. However, in the cell culture chip 1 of the present invention, the number of opening grooves (21, 22) connected to the recessed region 23 is not limited.

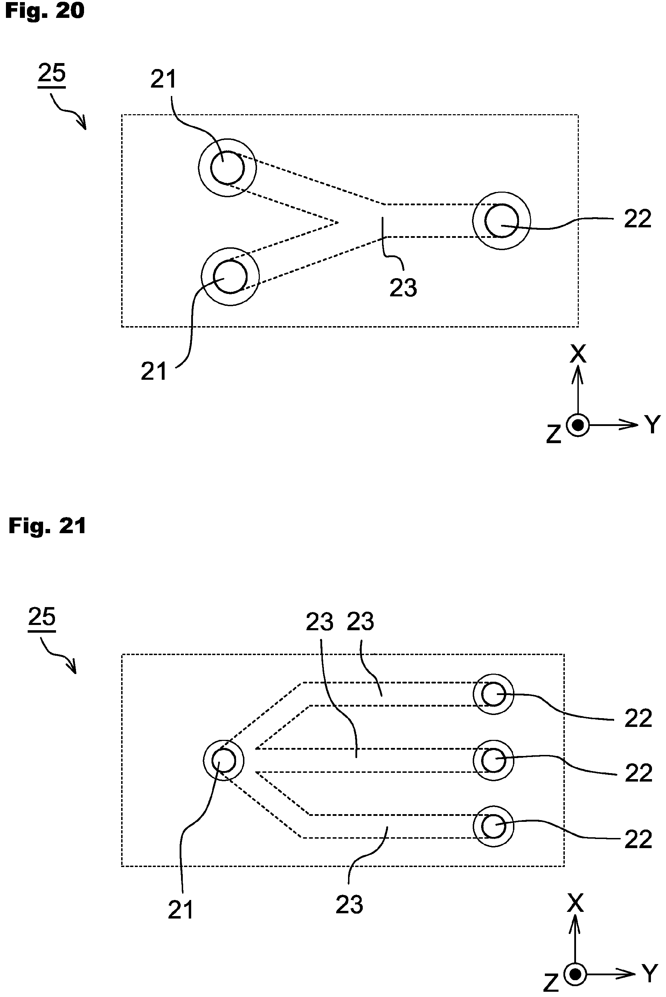

   Figs. 20 and 21 are plan views each illustrating, in the same manner as Fig. 4, a first portion 25 of a cell culture chip 1 according to other embodiments. In the cell culture chip 1 shown in Fig. 20, the first portion 25 includes two first opening grooves 21 and one second opening groove 22, and the opening grooves (21, 22) are connected by a recessed region 23. Further, in the cell culture chip 1 shown in Fig. 21, the first portion 25 includes one first opening groove 21 and three second opening grooves 22, and three recessed regions 23 are provided to connect the first opening groove 21 and the respective second opening grooves 22. The structures of the opening grooves (21, 22) and the recessed region 23 are the same as those of the above-described embodiments, and thus, the description thereof is omitted.
<2> The aspect of the number and arrangement of the first portions 25 (that is, the culture space) included in the cell culture chip 1 described in each of the above embodiments is merely an example. The number of first portions 25 arranged in each of the subdivisions 3 is not necessarily the same.

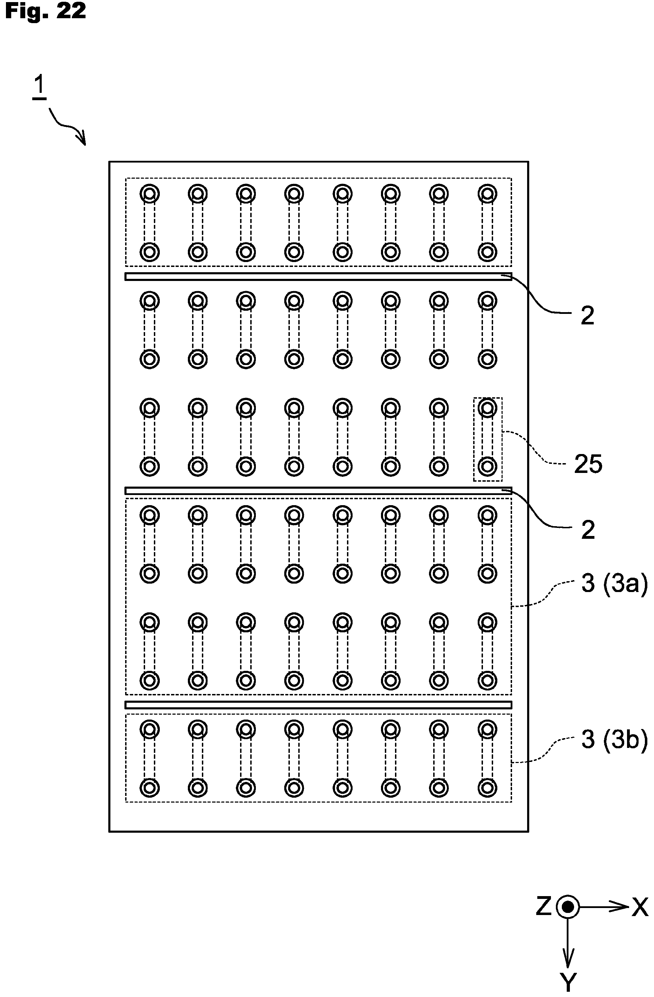

For example, as shown in Fig. 22, the cavities 2 extending in the X direction may be formed at three places spaced apart in the Y direction, the subdivision 3 (3b) in which eight first portions 25 are arranged may be formed outside the cavity 2 positioned outermost in the Y direction, and the subdivision 3 (3a) in which 16 (8 × 2 rows) first portions 25 are arranged may be formed in a region enclosed by the cavities 2 on both sides.

### DESCRIPTION OF REFERENCE SIGNS

- 1: Cell culture chip
- 2 (2c, 2d): Cavity
- 2a, 2b: End of cavity
- 3 (3a, 3b, 3c): Subdivision
- 10: Bottom substrate
- 10a: Surface of bottom substrate
- 20: Base body part
- 20a: First surface of base body part
- 20b: Second surface of base body part
- 21: First opening groove
- 22: Second opening groove
- 21f, 22f: Region where opening groove is to be formed
- 23: Recessed region
- 23f: Region where recessed region is to be formed
- 25: First portion (culture space forming region)
- 31: First side part
- 32: Second side part
- 35: Outer edge
- 36: Connecting part
- 37 (37a, 37b): Island part
- 38: Branch connecting part
- 40: Culture solution
- 41: Cell
- 60: Mold
- 61: Resin
- 62: Resin merging region
- 65: Gate
- 100: Conventional cell culture chip
- 101: Thin film
- 110: First substrate
- 110a: Surface of first substrate
- 120: Second substrate
- 120a, 120b: Surface of second substrate
- 121, 122: Opening
- 123: Recess

## Claims

1. A cell culture chip (1) comprising a bottom substrate (10) and a base body part (20) bonded onto the bottom substrate (10), wherein
the base body part (20) comprises:
a first surface (20a) and a second surface (20b) opposing the first surface (20a);
a plurality of first portions (25) configured to form a culture space and dispersedly arranged at a plurality of places in a direction parallel to the first surface (20a); and
a cavity (2) that penetrates the base body part (20) from the first surface (20a) and reaches the second surface (20b), in a region where the first portions (25) are not formed,
the plurality of first portions (25) each comprises:
a recessed region (23) formed to extend in the direction parallel to the first surface (20a) on a side of the first surface (20a); and
a plurality of opening grooves (21, 22) formed to penetrate the base body part (20) from a plurality of places in the recessed region (23) and reach the second surface (20b),
the cavity (2) is configured such that at least a part of an end in the direction parallel to the first surface (20a) is positioned inside an outer edge of the base body part (20), and
the bottom substrate (10) and the first surface (20a) of the base body part (20) are bonded together to form the culture space in which the recessed region (23) is sandwiched between the bottom substrate (10) and the base body part (20).

2. The cell culture chip (1) according to claim 1, wherein
the base body part (20) is constituted of a base body substrate having a rectangular shape and being a substrate different from the bottom substrate (10),
when viewed from a direction orthogonal to the first surface (20a), the plurality of first portions (25) are arranged in a matrix, respectively aligned in a first direction parallel to a first side part (31) constituting the outer edge of the base body part (20) and in a second direction parallel to a second side part (32) constituting the outer edge and being different from the first side part (31), and
the cavity (2) is formed to extend in the first direction at a position between regions where the plurality of first portions (25) are arranged in the first direction.

3. The cell culture chip (1) according to claim 2, wherein the cavity (2) is formed to extend in the first direction at a position outside, in the second direction, a region where the plurality of first portions (25) is arranged in the first direction.

4. The cell culture chip (1) according to claim 2 or 3, wherein the cavity (2) has one end in the first direction that reaches the first side part (31).

5. The cell culture chip (1) according to any one of claims 2 to 4, wherein
the plurality of opening grooves (21, 22) comprises a first opening groove (21) and a second opening groove (22) formed at a position spaced apart from the first opening groove (21) in the second direction, and
the recessed region (23) is formed to extend in the second direction to connect the first opening groove (21) to the second opening groove (22).

6. The cell culture chip (1) according to claim 1, wherein
the base body part (20) further comprises:
an outer edge (35) having a rectangular frame shape and including first side parts (31) facing each other and second side parts (32) facing each other;
a connecting part (36) that connects the first side parts (31) facing each other by extending in a second direction parallel to the second side parts (32); and
an island part (37) having a plate shape including the first surface (20a) and the second surface (20b), the island part (37) is provided in plural numbers and the plurality of the island parts (37) being dispersedly arranged at a plurality of positions between the connecting part (36) and the outer edge (35) in a state of being connected to a part of the connecting part (36),
the first portion (25) is formed in the island part (37), and
the cavity (2) is formed in a region enclosed by the outer edge (35) and the connecting part (36) at a position outside the island part (37).

7. The cell culture chip (1) according to claim 6, wherein
the base body part (20) further comprises a plurality of the [[first]] connecting parts (36) formed spaced apart in a first direction parallel to the first side part (31), and
the island part (37) is formed at a position enclosed by the connecting parts (36) adjacent to each other in the first direction.

8. The cell culture chip (1) according to claim 6 or 7, wherein
the plurality of opening grooves (21, 22) included in the first portion (25) formed in the island part (37) include a first opening groove (21) and a second opening groove (22) formed at a position spaced apart from the first opening groove (21) in the second direction, and
the recessed region (23) included in the first portion (25) formed in the island part (37) is formed to extend in the second direction so as to connect the first opening groove (31) to the second opening groove (22).

## Patentansprüche

1. Zellkulturchip (1), umfassend ein Bodensubstrat (10) und einen Grundkörperteil (20), der an das Bodensubstrat (10) gebunden ist, wobei
der Grundkörperteil (20) umfasst:
eine erste Oberfläche (20a) und eine zweite Oberfläche (20b), die der ersten Oberfläche (20a) gegenüberliegt,
eine Vielzahl von ersten Abschnitten (25), die dazu ausgebildet sind, einen Kulturraum zu bilden, und die verteilt an einer Vielzahl von Stellen in einer Richtung parallel zur ersten Oberfläche (20a) angeordnet sind, und
einen Hohlraum (2), der den Grundkörperteil (20) von der ersten Oberfläche (20a) aus durchdringt und die zweite Oberfläche (20b) in einem Bereich erreicht, in dem die ersten Abschnitte (25) nicht ausgebildet sind,
wobei die Vielzahl der ersten Abschnitte (25) jeweils umfasst:
einen ausgesparten Bereich (23), der so ausgebildet ist, dass er sich in der Richtung parallel zu der ersten Oberfläche (20a) auf einer Seite der ersten Oberfläche (20a) erstreckt, und
eine Vielzahl von Öffnungsnuten (21, 22), die so ausgebildet sind, dass sie den Grundkörperteil (20) von einer Vielzahl von Stellen in dem ausgesparten Bereich (23) durchdringen und die zweite Oberfläche (20b) erreichen,
wobei der Hohlraum (2) so ausgebildet ist, dass zumindest ein Teil eines Endes in der Richtung parallel zur ersten Oberfläche (20a) innerhalb eines Außenrandes des Grundkörperteils (20) angeordnet ist, und
das Bodensubstrat (10) und die erste Oberfläche (20a) des Grundkörperteils (20) miteinander verbunden sind, um den Kulturraum zu bilden, in dem der ausgesparte Bereich (23) zwischen dem Bodensubstrat (10) und dem Grundkörperteil (20) eingeschoben ist.

2. Zellkulturchip (1) nach Anspruch 1, wobei
der Grundkörperteil (20) aus einem Grundkörpersubstrat besteht, das eine rechteckige Form hat und ein von dem Bodensubstrat (10) verschiedenes Substrat ist,
wobei bei Betrachtung aus einer zur ersten Oberfläche (20a) orthogonalen Richtung die Vielzahl der ersten Abschnitte (25) in einer Matrix angeordnet ist, jeweils ausgerichtet in einer ersten Richtung parallel zu einem ersten Seitenteil (31), das den Außenrand des Grundkörperteils (20) bildet, und in einer zweiten Richtung parallel zu einem zweiten Seitenteil (32), das den Außenrand bildet und von dem ersten Seitenteil (31) verschieden ist, und
wobei der Hohlraum (2) so ausgebildet ist, dass er sich in der ersten Richtung an einer Position zwischen Bereichen erstreckt, in denen die Vielzahl der ersten Abschnitte (25) in der ersten Richtung angeordnet ist.

3. Zellkulturchip (1) nach Anspruch 2, wobei der Hohlraum (2) so ausgebildet ist, dass er sich in der ersten Richtung an einer Position in der zweiten Richtung außerhalb eines Bereichs erstreckt, in dem die Vielzahl der ersten Abschnitte (25) in der ersten Richtung angeordnet ist.

4. Zellkulturchip (1) nach Anspruch 2 oder 3, wobei der Hohlraum (2) ein Ende in der ersten Richtung hat, das das erste Seitenteil (31) erreicht.

5. Zellkulturchip (1) nach einem der Ansprüche 2 bis 4, wobei
die Vielzahl von Öffnungsnuten (21, 22) eine erste Öffnungsnut (21) und eine zweite Öffnungsnut (22) umfasst, die an einer von der ersten Öffnungsnut (21) in der zweiten Richtung beabstandeten Position ausgebildet ist, und
der ausgesparte Bereich (23) so ausgebildet ist, dass er sich in der zweiten Richtung erstreckt, um die erste Öffnungsnut (21) mit der zweiten Öffnungsnut (22) zu verbinden.

6. Zellkulturchip (1) nach Anspruch 1, wobei
der Grundkörperteil (20) ferner umfasst:
einen Außenrand (35), der eine rechteckige Rahmenform aufweist und einander gegenüberliegende erste Seitenteile (31) und einander gegenüberliegende zweite Seitenteile (32) umfasst,
einen Verbindungsteil (36), der die einander gegenüberliegenden ersten Seitenteile (31) miteinander verbindet, indem er sich in einer zweiten Richtung parallel zu den zweiten Seitenteilen (32) erstreckt, und
einen Inselteil (37) mit einer Plattenform, der die erste Oberfläche (20a) und die zweite Oberfläche (20b) umfasst, wobei der Inselteil (37) in Mehrzahl vorgesehen ist und die Vielzahl der Inselteile (37) an einer Vielzahl von Positionen zwischen dem Verbindungsteil (36) und dem Außenrand (35) in einem Zustand der Verbindung mit einem Teil des Verbindungsteils (36) verteilt angeordnet ist,
wobei der erste Abschnitt (25) in dem Inselteil (37) ausgebildet ist und
der Hohlraum (2) in einem Bereich ausgebildet ist, der von dem Außenrand (35) und dem Verbindungsteil (36) an einer Position außerhalb des Inselteils (37) umgeben ist.

7. Zellkulturchip (1) nach Anspruch 6, wobei
der Grundkörperteil (20) ferner eine Vielzahl der [[ersten]] Verbindungsteile (36) umfasst, die in einer zum ersten Seitenteil (31) parallelen ersten Richtung beabstandet ausgebildet sind, und
der Inselteil (37) an einer Position ausgebildet ist, die von den Verbindungsteilen (36) umgeben ist, die einander in der ersten Richtung benachbart sind.

8. Zellkulturchip (1) nach Anspruch 6 oder 7, wobei
die Vielzahl von Öffnungsnuten (21, 22), die in dem ersten Abschnitt (25) umfasst sind, der im Inselteil (37) ausgebildet ist, eine erste Öffnungsnut (21) und eine zweite Öffnungsnut (22) aufweist, die an einer von der ersten Öffnungsnut (21) in der zweiten Richtung beabstandeten Position ausgebildet ist, und
der ausgesparte Bereich (23), der in dem ersten Abschnitt (25) umfasst ist, der in dem Inselteil (37) ausgebildet ist, so ausgebildet ist, dass er sich in der zweiten Richtung erstreckt, um die erste Öffnungsnut (31) mit der zweiten Öffnungsnut (22) zu verbinden.

## Revendications

1. Puce de culture cellulaire (1) comprenant un substrat inférieur (10) et une partie corps de base (20) liée au substrat inférieur (10), dans laquelle
la partie corps de base (20) comprend :
une première surface (20a) et une deuxième surface (20b) opposée à la première surface (20a) ;
une pluralité de premières parties (25) configurées pour former un espace de culture et disposées de manière dispersée à une pluralité d'emplacements dans une direction parallèle à la première surface (20a) ; et
une cavité (2) qui pénètre dans la partie corps de base (20) depuis la première surface (20a) et atteint la deuxième surface (20b), dans une région où les premières parties (25) ne sont pas formées,
la pluralité de premières parties (25) comprenant chacune :
une région en retrait (23) formée de manière à s'étendre dans la direction parallèle à la première surface (20a) sur un côté de la première surface (20a) ; et
une pluralité de rainures d'ouverture (21, 22) formées de manière à pénétrer dans la partie corps de base (20) à partir d'une pluralité d'emplacements dans la région en retrait (23) et atteindre la deuxième surface (20b),
la cavité (2) est configurée de telle sorte qu'au moins une partie d'une extrémité dans la direction parallèle à la première surface (20a) est positionnée à l'intérieur d'un bord extérieur de la partie corps de base (20), et
le substrat inférieur (10) et la première surface (20a) de la partie corps de base (20) sont liés ensemble pour former l'espace de culture dans lequel la région en retrait (23) est prise en sandwich entre le substrat inférieur (10) et la partie corps de base (20).

2. Puce de culture cellulaire (1) selon la revendication 1, dans laquelle
la partie corps de base (20) est constituée d'un substrat de corps de base ayant une forme rectangulaire et étant un substrat différent du substrat inférieur (10),
vues depuis une direction orthogonale à la première surface (20a), la pluralité de premières parties (25) sont disposées en matrice, respectivement alignées dans une première direction parallèle à une première partie latérale (31) constituant le bord extérieur de la partie corps de base (20) et dans une deuxième direction parallèle à une deuxième partie latérale (32) constituant le bord extérieur et différente de la première partie latérale (31), et
la cavité (2) est formée de manière à s'étendre dans la première direction à une position située entre des régions où la pluralité de premières parties (25) sont disposées dans la première direction.

3. Puce de culture cellulaire (1) selon la revendication 2, dans laquelle la cavité (2) est formée de manière à s'étendre dans la première direction à une position située à l'extérieur, dans la deuxième direction, d'une région où la pluralité de premières parties (25) sont disposées dans la première direction.

4. Puce de culture cellulaire (1) selon la revendication 2 ou 3, dans laquelle la cavité (2) a une extrémité dans la première direction qui atteint la première partie latérale (31).

5. Puce de culture cellulaire (1) selon l'une quelconque des revendications 2 à 4, dans laquelle
la pluralité de rainures d'ouverture (21, 22) comprend une première rainure d'ouverture (21) et une deuxième rainure d'ouverture (22) formée à une position espacée de la première rainure d'ouverture (21) dans la deuxième direction, et
la région en retrait (23) est formée de manière à s'étendre dans la deuxième direction pour relier la première rainure d'ouverture (21) à la deuxième rainure d'ouverture (22).

6. Puce de culture cellulaire (1) selon la revendication 1, dans laquelle
la partie corps de base (20) comprend en outre :
un bord extérieur (35) ayant une forme de cadre rectangulaire et incluant des premières parties latérales (31) se faisant face et des deuxièmes parties latérales (32) se faisant face ;
une partie de liaison (36) qui relie les premières parties latérales (31) se faisant face en s'étendant dans une deuxième direction parallèle aux deuxièmes parties latérales (32) ; et
une partie îlot (37) ayant une forme de plaque incluant la première surface (20a) et la deuxième surface (20b), la partie îlot (37) étant prévue en plusieurs exemplaires et la pluralité de parties îlot (37) étant disposées de manière dispersée à une pluralité de positions entre la partie de liaison (36) et le bord extérieur (35) dans un état où elles sont reliées à une partie de la partie de liaison (36),
la première partie (25) est formée dans la partie îlot (37), et
la cavité (2) est formée dans une région délimitée par le bord extérieur (35) et la partie de liaison (36) à une position située à l'extérieur de la partie îlot (37).

7. Puce de culture cellulaire (1) selon la revendication 6, dans laquelle
la partie corps de base (20) comprend en outre une pluralité de [[premières]] parties de liaison (36) formées à distance les unes des autres dans une première direction parallèle à la première partie latérale (31), et
la partie îlot (37) est formée à une position délimitée par les parties de liaison (36) adjacentes les unes aux autres dans la première direction.

8. Puce de culture cellulaire (1) selon la revendication 6 ou 7, dans laquelle
la pluralité de rainures d'ouverture (21, 22) incluses dans la première partie (25) formée dans la partie îlot (37) inclut une première rainure d'ouverture (21) et une deuxième rainure d'ouverture (22) formée à une position espacée de la première rainure d'ouverture (21) dans la deuxième direction, et
la région en retrait (23) incluse dans la première partie (25) formée dans la partie îlot (37) est formée de manière à s'étendre dans la deuxième direction afin de relier la première rainure d'ouverture (31) à la deuxième rainure d'ouverture (22).
